# EUROPEAN PATENT SPECIFICATION

(11) **EP 4 447 796 B1**
(45) Date of publication and mention of the grant of the patent: **04.06.2025**
(21) Application number: 22844557.3
(22) Date of filing: 30.12.2022
(51) Int. Cl.: A61B 5/024, A61B 5/145, A61B 5/00

(54) **INTEGRATION SYSTEM OF SENSING CONSUMABLES FOR WEARABLE DEVICES**
TRAGBARE VORRICHTUNG ZUR KONTINUIERLICHEN ÜBERWACHUNG VON GESUNDHEITSPARAMETERN
DISPOSITIF PORTABLE POUR LA SURVEILLANCE CONTINUE DE PARAMÈTRES DE SANTÉ

(30) Priority: 30.12.2021 EP 21383241
(43) Date of publication of application: 23.10.2024
(73) Proprietor: Onalabs Inno-Hub, 08290 Cerdanyola del Valles (ES)
(72) Inventor: RABOST GARCÍA, Genís, 08290 Cerdanyola del Valles (ES); MUÑOZ PASCUAL, Francesc Xavier, 08290 Cerdanyola del Valles (ES); AGUILAR TORÁN, Javier, 08290 Cerdanyola del Valles (ES); PUNTER VILLAGRASA, Jaime, 08290 Cerdanyola del Valles (ES); COLMENA RUBIAL, Valeria, 08290 Cerdanyola del Valles (ES)
(74) Representative: Hoffmann Eitle
(86) International application number: PCT/EP2022/088083
(87) International publication number: WO 2023/126524

(56) References cited:
- EP-A1- 2 641 533
- JP-A- 2019 129 965
- US-A1- 2016 278 672
- US-A1- 2020 312 453
- US-A1- 2020 397 315

## Description

### Field and object of the invention

The present invention belongs to the technical filed of wearable medical devices both for clinical and sport applications.

An object of the invention is the provision of a wearable device for monitoring of health condition of patients or sport persons, without blood extraction, that can be used for a large variety of applications, and which can be used in a simple and intuit manner.

The invention can advantageously be used in sports medicine and/or sports health sectors for remote effort and/or fatigue assessment.

### Background of the invention

There are many types of wearable devices that can be worn by a user to continuously monitor an individual's activities, such as walking or running without unduly interrupting or limiting those activities. These wearable devices include electronics, and physiological sensors configured to sense certain physiological parameters of the wearer, such as heart rate, as well as motion sensors, GPS, etc.

These known devices generally have the same format, and are adapted to monitor only one physiological parameter of the wearer, and have limited capacities in terms of electronic processing, and communications capacities.

Moreover, when these sensors are directed to measure sweat-related variables, there is an added difficulty due to past sweat droplets interfering with current ones, leading to erroneous ones.

There is therefore an unmet need for a wearable device capable of monitoring more than one physiological parameter of the wearer including a sweat related parameters such that the measurements are unsusceptible to erroneous readings due to past sweat samples.

US 2020/397315 A1 discloses a system for collecting and analysing sweat which includes a sweat sensing device having at least one sensor configured to sense one of physiologic information, biologic information, biochemical information, and biometric information, from skin of a person to whom the sweat sensing device is attached. A wireless transmitter is mounted to the sweat sensing device and electrically connected to the at least one sensor. An interactive console is configured to produce a real time feedback to an operator of the system for collecting and analysing sweat, includes a wireless receiver, and is in wireless communication with the sweat sensing device. The interactive console is further configured to receive data from the at least one sensor, display the data received from the at least one sensor, adjust an environmental condition based on the sensor data received, and display the adjustment to the environmental condition.

US 2016/278672 A1 discloses a wearable electronic device including a body portion wearable on a human body, a circuit portion mounted on the body portion, and a sensor portion contacting the human body to measure a bio signal, wherein the sensor portion is detachably coupled to the body portion.

EP 2 641 533 A1 discloses a sensor system and a sensor system using method which improve operability of a placing operation of placing a sensor in a living body and improve user-friendliness of users. The sensor system has: a sensor member which has the sensor which detects analyte components of the living body and transmits a signal, and a retaining portion which retains the sensor; a needle assembly which is detachably attached to the sensor member and which has a guide needle which guides insertion of the sensor to the living body; a signal processing circuit member which is detachably attached to the sensor member, and which has an electronic circuit which processes the signal from the sensor; and a fixing member which fixes the sensor member to a skin of the living body, and the sensor is placed in the living body in a state in which the signal processing circuit member is attached to the sensor member.

JP 2019 129965 A discloses a perspiration amount measuring device includes a perspiration sensor device which is closely attached to a part of a body of a subject for measuring a perspiration amount. The perspiration sensor device includes a case part with a storage space and an opening being formed at a part of the case part and communicating with the inside and the outside, through which moisture passes. An adsorbent is provided, which is detachably put into the storage space of the case part and covers the opening. A pair of electrodes are provided, which is located in contact with the adsorbent in the case part for electrically measuring an amount of the moisture adsorbed by the adsorbent. The pair of the electrode is connected to a measuring part body for calculating the perspiration amount, and measure the perspiration amount by measuring and calculating an electric value between the pair of the electrodes. An annular convex part is provided to a peripheral edge part outside the opening.

### Summary of the invention

The invention, which is defined in claim 1, refers to a wearable device for continuous monitoring of health parameters of a user, that comprises three main parts, namely: a housing, means for attaching the housing to a part of the user's body, and a fungible or consumable component configured to be manually attachable and detachable from the housing, and that can be disposed after its use.

The consumable component comprises: a sweat collection inlet for collecting sweat when the device is worn by the user, at least one sensor for measuring a sweat biomarker, and a microfluidic channel for conveying collected sweat from the inlet to the sweat sensor.

Preferably, the consumable component has a sensing chamber communicated with the sweat inlet through the microfluidic channel. The sensing chamber encloses at least one of the following sensors: a sweat lactate sensor, a sweat conductivity sensor, a metabolites sensor, an ions sensor, and/or an amino acids sensor.

The advantage of this format of wearable device formed by a permanent part (the housing with the electronics of the device), and a consumable part with the sensors that can be disposed after its use, is that the same device can be used for a large variety of health monitoring applications, simply by providing a set of consumables each one provided with the sensors required for each particular application.

In addition, the wearable device comprises a processing unit enclosed in the housing and adapted for processing data provided by the sensor or sensors, and electric connection means for electrically connecting the sensor or sensors with the processing unit, when the consumable component is operatively attached to the main housing.

Preferably, the wearable device includes a communication module enclosed in the housing, and adapted for the wireless transmission of data processed and calculated by the processing unit, to an external device such as: a smart phone, a smart watch, etc.

The housing has a front surface and a back surface, and the consumable component is attachable to the back surface of the housing, in a way that the consumable component is overlapped with the back surface of the housing when they are operatively attached.

In turn, the consumable component has a contact surface provided to be in contact with the user's skin when the consumable component is operatively attached to the housing and the housing is attached to a part of the user's body. The sweat collection inlet is provided on the consumable component's contact surface, to collect sweat from the user's skin.

The housing has a cavity at its back surface for receiving a battery for supplying power to the processing unit and to other electronics of the device, and a lid for closing the cavity and enclosing the battery therein, so that for replacing the battery the lid has to be removed.

The device is configured such that the consumable component is placed over the lid, when the consumable component is operatively attached to the housing.

The means for attaching the housing to a part of the user's body, are preferably implemented as a flexible band or strap having two ends respectively couplable with the housing. Preferably, the flexible band is fitted with at least one biosensor for measuring a vital-sign or physiological sign of the user, when the flexible band is attached to a part of the user's body such as an arm, wrist, chest etc., thus, in addition to the sweat measurements, a vital sign or signs measurements are also taken in direct contact with the skin, with the same de device, and simultaneously while sweat parameters are measured. The biosensor or biosensors are selected from the following list: a heart rate sensor, a respiratory rate sensor, blood pressure sensor, body temperature sensor, and oxygen saturation sensor.

Alternatively, the means for attaching the main housing to a part of a user's body, comprises an adhesive surface suitable to be adhered on a user's skin. For example, this adhesive surface is provided on a surface of the consumable component meant to be in contact with the user's skin.

The device has a pair of connectors for mechanically connecting the flexible band with the housing, and for electrically connecting the biosensors with the processing unit.

Preferably, the consumable component is generally a flat body, in the form of a card, so that it does not protrude from the housing when they are coupled.

A part or area of the back surface of the housing is generally flat, and the consumable component is attachable and detachable from the housing by moving the consumable component on a plane coplanar with said generally flat part of the back surface. This way of coupling the consumable component with the housing, provides the advantage that it is easy and intuitive for users to attach and detach both parts even when the device is worn by a user.

In a preferred embodiment, for attaching the consumable component to the housing, the housing has at its back surface a pair of guides opposite each other, and the consumable component has a pair of sides wings dimensioned and configured such that the consumable component fits inside the space defined in between the pair of guides. The consumable component is attachable and detachable from the housing, by sliding its side wings on the guides and by moving the consumable component on the back surface of the housing.

When coupled, the consumable component is retained firmly attached to the housing, while the user is running or working out. In addition, when coupled, the pair of guides are configured to operatively couple the electric connection means of the consumable component in a sealed way.

It would be understood by a skilled person in the art, that the means for attaching and detaching the consumable component from the housing, can be embodied in many different ways within the scope of this invention.

Preferably, the consumable component further comprises a sweat volume sensor for measuring volume of the collected sweat, and the processing unit is adapted for receiving and processing data provided by the sweat biomarker sensor, the vital-sign biosensor of the flexible band, and the sweat volume sensor.

The sweat volume sensor comprises a microfluidic circuit or a microfluidic reservoir, fluidly communicated with the sensing chamber and arranged downstream the sensing chamber, and a pair of electrodes such that the microfluidic reservoir is arranged in between the two electrodes, in a way that a capacitance value between the two electrodes is variable depending on the amount of sweat in the reservoir. The two electrodes are a pair of conductive strips opposite each other, and more preferably the electrodes are embodied as conductive flexible strips.

The microfluidic reservoir can have any form, for example the microfluidic reservoir can be a straight or curved conduit, or it can be formed as a conduit having a meander configuration.

In a preferred embodiment, the sweat sensor is a sweat lactate sensor, and the vital-sign sensor is a heart rate sensor, and wherein the processing unit is further adapted to calculate or estimate, preferably by means of machine learning algorithms, a blood lactate concentration based on data provided by: the sweat lactate sensor, the sweat volume sensor and heart rate sensor. This data received by the processing unit is in the form of electric signals.

In summary, some of the main advantages of the invention are the followings:
- the format of the wearable device, is already used by athletes (heart rate monitor) to which they are accustomed in their regular training;
- the permanent part of the device (housing) can be used by itself, in case that chemical measurement with the consumable part is not necessary (already known training routine, etc...);
- the permanent part (housing) is compatible with different types of consumable parts depending on the parameters to be measured, such that the device is an useful tool not only for the end user but also for the clinic/sports physician, who can adapt the device to the requirements of the patient/study;
- simple to use by insertion of the consumable that guarantees placement in the same place every time, limiting the variability that the user's own operation may entail;
- it allows to have more advanced electronics with advanced functionalities compared to patch wearables such as battery management, data processing, flash memory in case there is no possibility of communicating the data;
- the consumable part might have an adhesive surface to enhance its attachment with the permanent part.

The main application of the invention is focused on sports medicine or sports health sector, for example for monitoring dehydration monitoring in athletes, but depending on the combination of sensors used, the product can be oriented to different applications.

The present invention also relates to a wearable device for continuous monitoring of health parameters of a user, comprising: a housing having a front surface and a back surface, and a processing unit enclosed therein, means for attaching the housing to a part of the user's body, a consumable component configured to be manually coupled and uncoupled with the housing, and having a contact surface provided to be in contact with the user's skin when the consumable component is operatively coupled with the housing and the housing is attached to a part of the user's body, and wherein the consumable component comprises: a sweat collection inlet formed in the consumable component's contact surface, for collecting sweat when the device is worn by the user, at least one sensor for measuring a sweat biomarker, a microfluidic channel for conveying collected sweat from the inlet to the sweat sensor.

The wearable device further comprises: electric connection means for electrically connecting the sensor with the processing unit when the consumable component is operatively coupled with the housing. The processing unit is adapted for processing data provided by the sensor, and the coupling surfaces of the pair of guides and the consumable component are configured to operatively couple in a sealed way so that they seal the electrical connection.

In a more preferred embodiment, the surface of the consumable component configured to contact the surface of the pair of guides comprising the electrical connection means comprises an adhesive surface configured to further seal the coupling.

### Brief description of the drawings

Preferred embodiments of the invention are henceforth described with reference to the accompanying drawings, wherein:
Figure 1.- shows a perspective view of a preferred embodiment of the invention including an attachment band or strap.
Figure 2.- shows a perspective view of the main housing and the consumable part partially coupled together.
Figure 3.- shows another perspective view of the main housing from above, and part of the band ends.
Figure 4.- shows a front elevational view of the main housing.
Figure 5.- shows a perspective view of the main housing from below.
Figure 6.- shows an exploded view of the main housing components.
Figure 7.- shows in Figure A a perspective view of the main housing from below. In Figure B with the consumable part partially coupled, and in Figure C with the consumable part fully coupled.
Figure 8.- shows an schematic representation of the sweat volume sensor. The figure represents four stages (A - B) of filling of the microfluidic channel.
Figure 9.- shows a perspective view of the consumable device from the face opposite the surface meant to be in contact with the skin.
Figure 10.- shows a schematic representation in plan view of the consumable device.
Figure 11.- shows a cross-sectional view of the consumable device, taken at cutting plane A-A in figure 10.
Figure 12.- shows another cross-sectional view of the consumable device, taken at cutting plane B-B in figure 10.

### Preferred embodiment of the invention

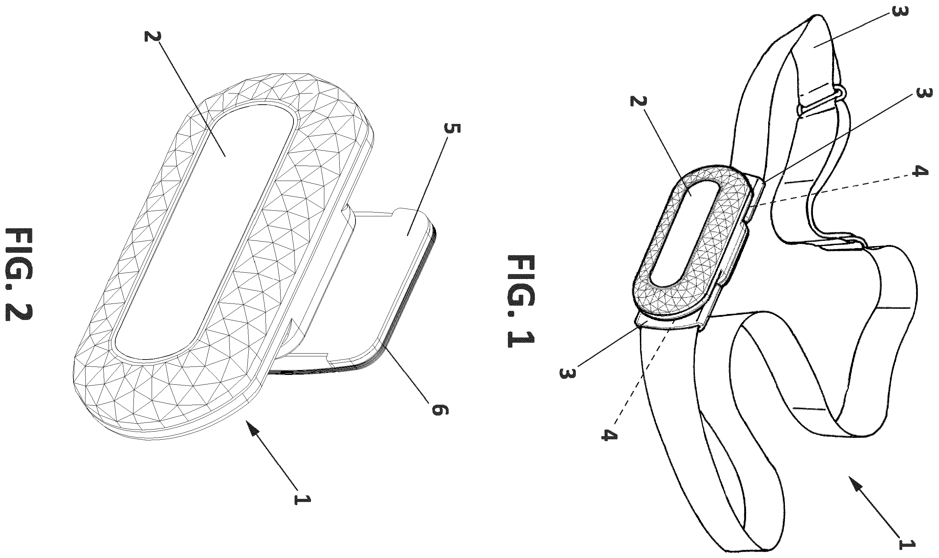

**Figure 1** shows an exemplary embodiment of a wearable device (1) according to the invention comprising a housing (2) and a flexible band or strap (3) for attaching the main housing to a part of a user's body, and a pair of electric and mechanic connectors or snap buttons (4) provided in the housing (2) for mechanically and electrically connecting the flexible band ends with the housing (2).

The flexible band (3) is implemented as an elastic textile tape with female connectors at its ends to be coupled with the snap buttons (4). The flexible band (3) is fitted conventionally with at least one biosensor for measuring a vital-sign or physiological sign of the user, in a known manner, for example a pair of electrodes to measure heart rate. The electrodes are made of bioelectric silicone to capture the electrocardiogram signal and extract the heart rate, and they have sufficient conductivity to obtain a quality heart rate signal and are resistant to continuous use and washing.

As shown more clearly in **Figure 2****,** the device (1) includes a consumable component (5) configured to be manually attached and detached from the housing (2), and having a contact surface (6) provided to be in contact with the user's skin when the consumable component (5) is coupled with the housing (2), and the housing (2) is attached to a user's body by means of the flexible band (3).

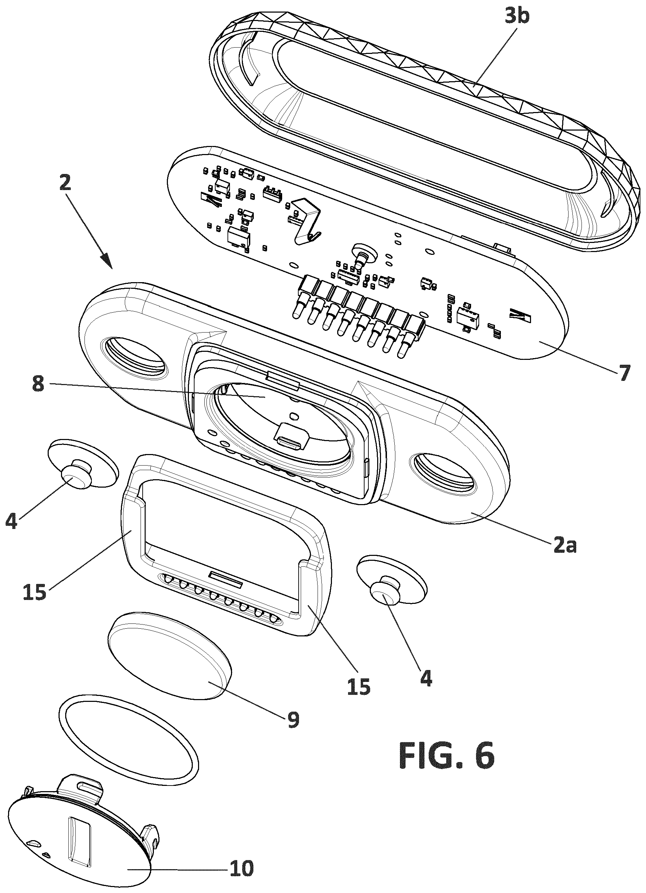

As shown in **Figure 6****,** the housing (2) is formed by two couplable parts, a base (2a) and a cover (2b) that configure a space when they are coupled within which an electronic circuit (7) is enclosed. This electronic circuit (7) implements the processing unit, sensors instrumentation, battery management, data processing and communication module for the wireless transmission of data processed by the processing unit.

The housing (2) in particular the base (2a) has a recess or cavity (8) for receiving a battery (9) for supplying power to the electronic circuit (7), and a lid (10) for closing and opening the cavity (8).

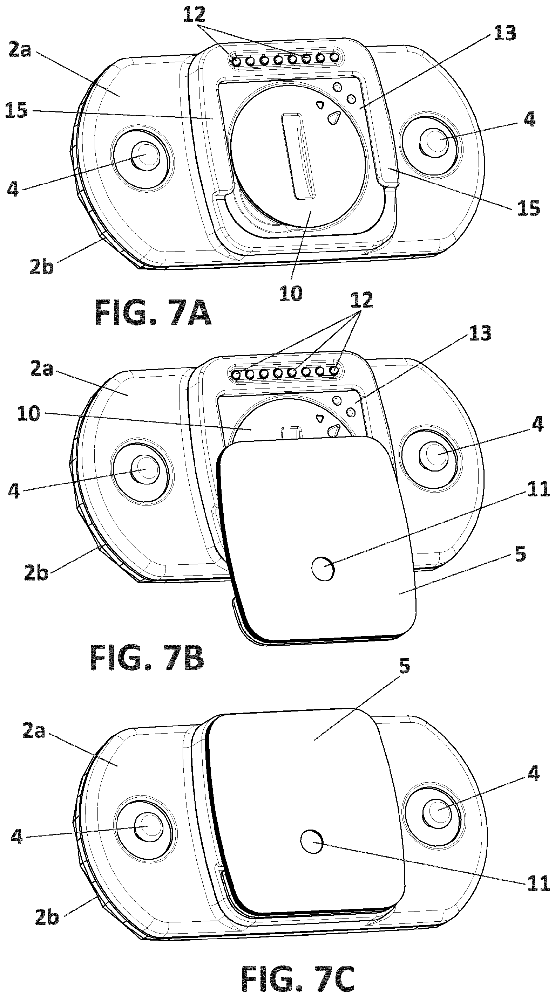

As represented in **Figures 2****,** **7B** and **7C****,** the consumable component (5) is generally a flat body, and the housing (2) and the consumable component (5) are configured, such that the consumable component (5) is couplable with the housing (2) by moving the consumable component (5) on a plane coplanar to the generally flat back surface (19) of the housing (2).

With this arrangement and as shown in **Figure 7C****,** the consumable component (5) is placed over lid (10) when is fully attached to the housing (2), and the lid (10) can only be accessed when the consumable component (5) is taken out from the housing (2).

A set of electric connectors (12) are provided at the back surface (13) of the base (2a) of the housing (2), in order to electrically connect the sensors fitted in the consumable component (5) with the processing unit (7). These connectors (12) are known spring-biased connectors, that stablish electric contact with corresponding electric pads (17) (provided in the consumable component (5), in a known manner when this is coupled with the housing (2).

The consumable component (5) can be mechanically and electrically coupled and uncoupled from the housing (2), in a quick and intuitive manner for a user, while ensuring functionality and good electrical connection with the housing (2).

The housing (2) has a back surface (13) provided with a pair of guides (15) opposite each other, in a way that a space or pocket is formed in between the guides (15) for receiving the consumable component (5) therein. In turn, the consumable component (5) has a pair of sides wings (16), dimensioned and configured to fit in the space formed in between the guides (15), such that the consumable component (5) is attachable to the housing (2) by inserting its side wings (16) respectively in the guides (15), and by moving the consumable component on the back surface (13) of the housing (2), as represented in the sequence of **Figures 7A, 7B,** and **7C****.**

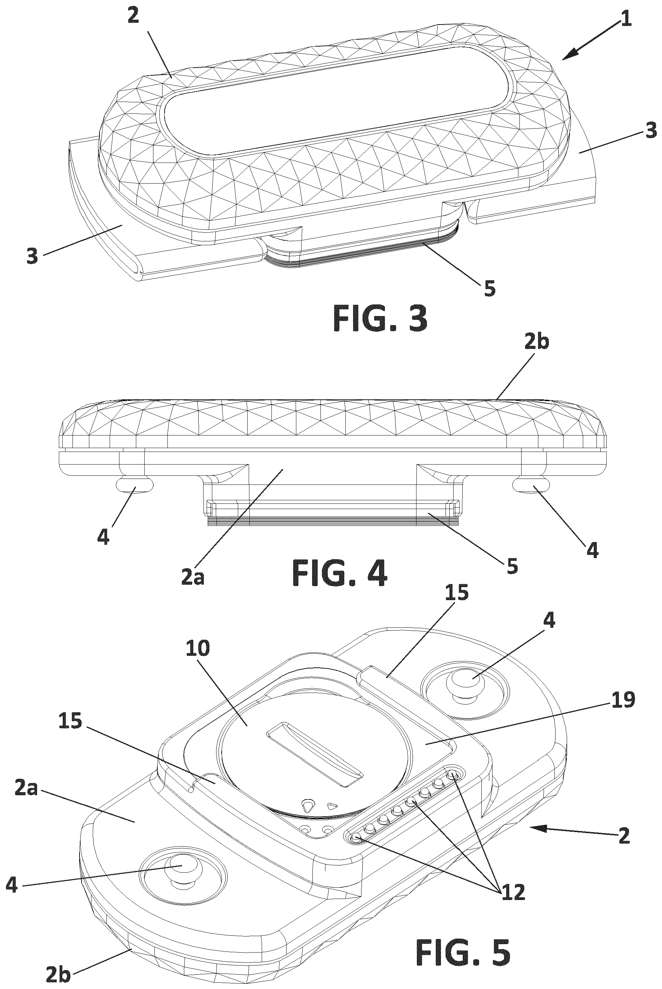

As shown In **Figures 5****,** **6****,** **7A, 7B,** and **7C****,** the pair of guides (15) also comprises the set of electric connectors (12), and as the pair of guides (15) provide an elevated surface, they can adequately comprise the set of electric connectors (12) such that they are at most at the same distance to the back surface (13) as the pair of guides (12). This enables that when the consumable component (5) is attached to the housing (2), the set of electric connectors (12) stablish electric contact with corresponding electric pads (17) in a sealed way. Therefore, the electrical contact is established so sweat cannot affect the readings, as it is comprised between the surface of the consumable component (5) and the surface of the pair of guides (15), such that sweat cannot reach the electrical contact location. It may be noted that the mere connection being tight enough, provides the seal that avoids sweat coming into contact with the electrical connections between the set of electric connectors (12) and the corresponding electric pads (17).

Advantageously, this allows for a simple integration system that does not require moving elements, nor coupling systems that may be prone to a wrong manual coupling, for example using magnets, that can still provide a sealed electrical connection between the housing (2) and the consumable component (5). This is essential for many application such as in sport use, wherein the amount of sweat and/or water present around the consumable component (5) is high.

In a more preferred embodiment, the surface of the consumable component (5) comprising the electric pads (17) and which is configured to contact the surface of the pair of guides (15) comprising the set of electric connectors (12), comprises an adhesive surface configured to further seal the coupling between the consumable component (5) and the housing (2). Advantageously, this allows for a system, wherein the housing (2) which is permanent, can have a simple coupling integration surface, and the consumable component (5), which is renewed each time, comprises the additional sealing elements that are prone to wearing from its use. Therefore, the system is configured to ensure that the electrical connection between the housing (2) and the consumable component (5) will be always sealed. This is of special importance in some environments, wherein there is little to no space to seal error, such as in aquatic environments.

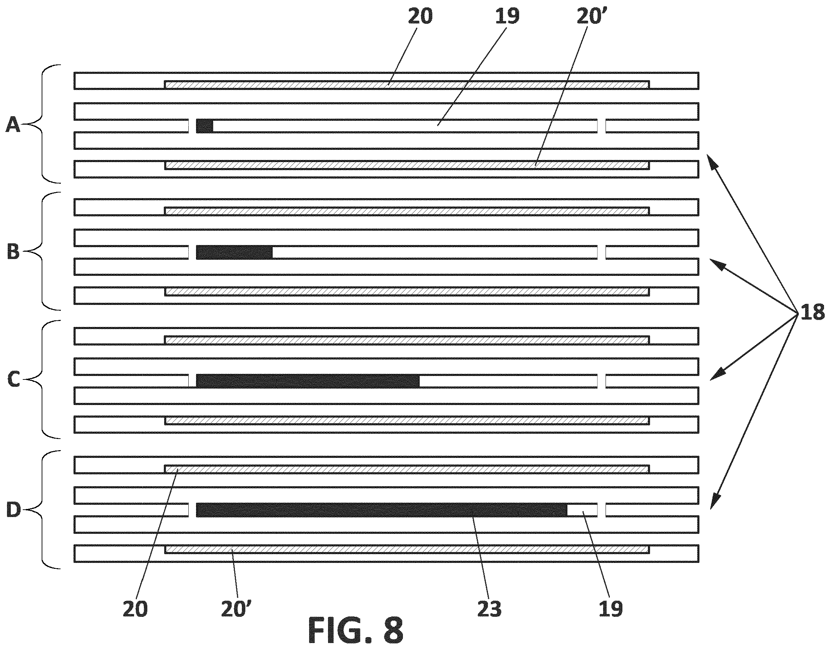

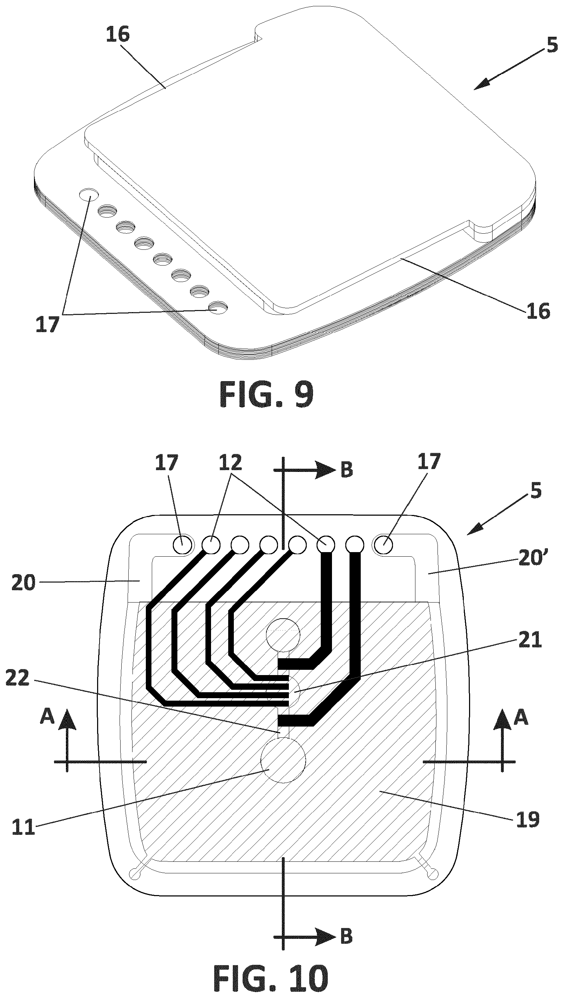

The consumable component (5) has a sweat volume sensor (18) represented schematically in **Figure 8****,** which comprises a microfluidic circuit or a microfluidic reservoir (19), fluidly communicated with a sensing chamber (21) and arranged downstream the sensing chamber (21), such that sweat would enter the inlet (11), and flow along the microfluidic channel (22) and the sensing chamber (21) to gradually fill the reservoir (19), as more clearly represented in **Figure 10****.**

In addition, the sweat volume sensor (18) comprises a pair of electrodes (20,20') and the microfluidic reservoir (19) arranged in between the two electrodes, such that a capacitance value between the two electrodes is variable depending on the amount of sweat in the reservoir.

The consumable component (5) can be manufactured as a stack of layers of plastic materials where the microfluidic channels and sensing chamber are formed by laser cutting or die cutting. The integrated sensors are electrochemical in nature, and therefore require electrodes that are fabricated by screen printing.

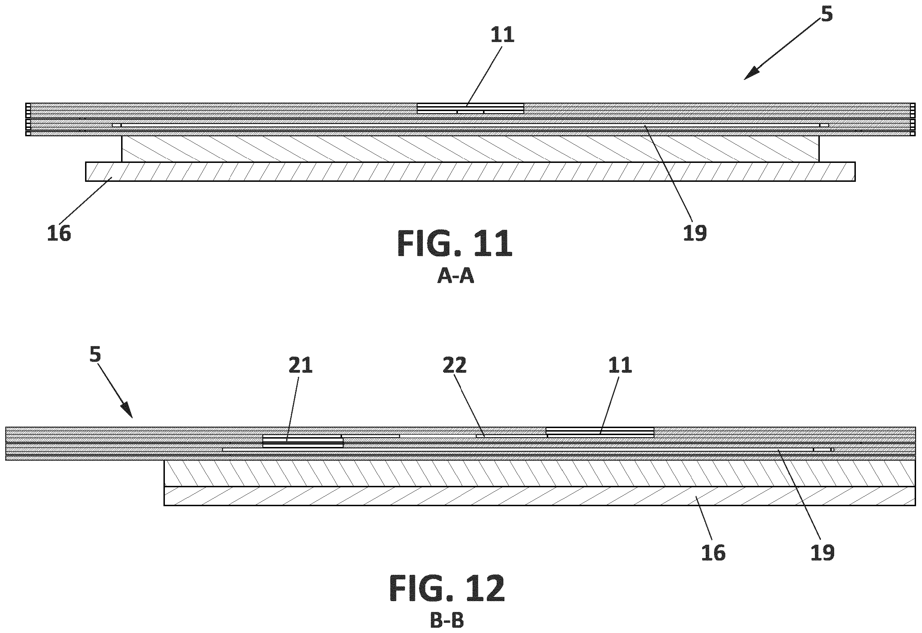

As shown in **Figures 11** and **12****,** in this particular embodiment, the sweat inlet (11), the microfluidic channel (22) and the sensing chamber (21), are placed above the microfluidic reservoir (19).

## Claims

1. A wearable device (1) for continuous monitoring of health parameters of a user, comprising:
a housing (2) having a front surface and a back surface (13), and a processing unit enclosed therein,
means (3) for attaching the housing (2) to a part of the user's body,
a consumable component (5) configured to be manually coupled and uncoupled with the housing (2), and having a contact surface (6) provided to be in contact with the user's skin when the consumable component (5) is operatively coupled with the housing (2) and the housing (2) is attached to a part of the user's body, and
wherein the consumable component (5) comprises:
a sweat collection inlet (11) formed in the consumable component's contact surface (6), for collecting sweat when the device (1) is worn by the user,
at least one sensor for measuring a sweat biomarker,
a microfluidic channel (22) for conveying collected sweat from the inlet (11) to the sweat sensor,
**characterised in that** the wearable device (1) further comprises:
electric connection means (12) for electrically connecting the sensor with the processing unit, when the consumable component (5) is operatively coupled with the housing (2),
wherein the processing unit is adapted for processing data provided by the sensor,
wherein the housing (2) has a pair of guides (15) opposite each other, and wherein the consumable component (5) has a pair of sides wings (16), and wherein the housing (2) and the consumable part are configured, such that the consumable component (5) is couplable with the housing (2) by inserting its side wings (16) respectively in the guides (15) and by moving the consumable component (5) on the back surface (13) of the housing (2),
wherein the electric connection means (12) are comprised within the coupling surfaces of the pair of guides (15) and the consumable component (5),
wherein the coupling surfaces of the pair of guides (15) and the consumable component (5) are configured to operatively couple in a sealed way so that they seal the electrical connection and
wherein the surface of the consumable component (5) configured to contact the surface of the pair of guides (15) comprising the electrical connection means (12) comprises an adhesive surface configured to further seal the coupling.

2. A wearable device (1) according to any of the preceding claims, wherein a part of the back surface (13) of the housing (2) is generally flat, and wherein the consumable component (5) is couplable and un-couplable with the housing (2) by moving the consumable component (5) on a plane parallel to said generally flat part of the back surface (13), or on a plane coplanar with the flat surface.

3. A wearable device (1) according to any of the preceding claims, wherein the consumable component (5) is generally a flat body.

4. A wearable device (1) according to any of the preceding claims, wherein the means (3) for attaching the main housing (2) to a part of a user's body, comprises a flexible band having two ends respectively couplable with the housing (2).

5. A wearable device (1) according to any of the claims 1 to 3, wherein the means (3) for attaching the main housing (2) to a part of a user's body, comprises an adhesive surface suitable to be adhered on a user's skin, and wherein preferably the adhesive surface is provided on the consumable component's contact surface (6).

6. A wearable device (1) according to any of the preceding claims, wherein housing (2) has a cavity (8) at its back surface (13) for receiving a battery (9) for supplying power to the processing unit, and a lid (10) for closing the cavity (8) and enclosing the battery (9) therein, and wherein the device (1) is configured such that the consumable component (5) is overlapped with the lid (10), when the consumable component (5) is operatively coupled with the housing (2).

7. A wearable device (1) according to claims 4 or 6 when dependent on claim 4, wherein the housing (2) is provided by a pair of electric connectors (12), and each end of the flexible band is fitted with metallic connectors for mechanically and electrically connecting the flexible band and the biosensor with the pair of electric connectors (12) of the housing (2).

8. A wearable device (1) according to claim 4, wherein the flexible band is provided with at least one biosensor arranged for measuring a vital-sign or physiological sign of the user, when the device (1) is worn by a user, and wherein the biosensor is selected from the following list: a heart rate sensor, a respiratory rate sensor, blood pressure sensor, body temperature sensor, and oxygen saturation sensor.

9. A wearable device (1) according to any of the preceding claims, wherein the consumable component (5) further comprises a sweat volume sensor (18) for measuring volume of the collected sweat, and wherein the processing unit is adapted for receiving and processing data provided by the sweat sensor, the vital-sign biosensor and the sweat volume measuring device.

10. A wearable device (1) according to any of the preceding claims, further comprising a communication module enclosed in the housing (2), and adapted for the wireless transmission of data processed by the processing unit.

11. A wearable device (1) according to claim 9, wherein the sweat sensor is a sweat lactate sensor, and the vital-sign sensor is a heart rate sensor, and wherein the processing unit is further adapted to calculate or estimate, preferably by means of machine learning algorithms, a blood lactate concentration based on data provided by: the sweat lactate sensor, the sweat volume sensor (18) and heart rate sensor.

12. A wearable device (1) according to any of the preceding claims, further comprising sensing chamber (21) and at least one of the following sensors: a sweat lactate sensor, a sweat conductivity sensor, metabolites sensor, ions sensors, amino acids sensor, and, placed in the sensing chamber (21), and wherein the microfluidic channel (22) communicates the sweat inlet (11) with the sensing chamber (21).

13. A wearable device (1) according to any of the preceding claims, wherein the sweat volume sensor (18) comprises a pair of electrodes (20, 20') and a microfluidic reservoir (19) in between the pair of electrodes (20, 20'), and fluidly communicated with the sensing chamber (21) and arranged downstream the sensing chamber (21), such that a capacitance value between the two electrodes (20, 20') is variable depending on the amount of sweat in the reservoir (19).

## Patentansprüche

1. Tragbare Vorrichtung (1) zur kontinuierlichen Überwachung von Gesundheitsparametern eines Benutzers, umfassend:
ein Gehäuse (2), welches eine Vorderfläche und eine Hinterfläche (13), und eine darin eingeschlossene Verarbeitungseinheit aufweist,
Mittel (3) zum Befestigen des Gehäuses (2) an einem Teil des Körpers des Benutzers,
eine Verbrauchskomponente (5), welche dazu ausgebildet ist, manuell mit dem Gehäuse (2) gekoppelt und entkoppelt zu werden, und welche eine Kontaktfläche (6) aufweist, welche dazu vorgesehen ist, der Haut des Benutzers in Kontakt mit zu sein, wenn die Verbrauchskomponente (5) mit dem Gehäuse (2) wirksam gekoppelt ist und das Gehäuse (2) an einem Teil des Körpers des Benutzers befestigt ist, und
wobei die Verbrauchskomponente (5) Folgendes umfasst:
einen Schweißsammeleinlass (11), welcher in der Kontaktfläche (6) der Verbrauchskomponente gebildet ist, um Schweiß zu sammeln, wenn die Vorrichtung (1) vom Benutzer getragen wird,
mindestens einen Sensor zum Messen eines Schweißbiomarkers,
einen mikrofluidischen Kanal (22) zum Fördern des gesammelten Schweißes vom Einlass (11) zum Schweißsensor,
**dadurch gekennzeichnet, dass** die tragbare Vorrichtung (1) zusätzlich Folgendes umfasst:
elektrische Anschlussmittel (12) zum elektrischen Anschließen des Sensors an die Verarbeitungseinheit, wenn die Verbrauchskomponente (5) mit dem Gehäuse (2) wirksam gekoppelt ist,
wobei die Verarbeitungseinheit dazu angepasst ist, vom Sensor bereitgestellte Daten zu verarbeiten,
wobei das Gehäuse (2) ein Paar einander gegenüberliegende Führungen (15) aufweist, und wobei die Verbrauchskomponente (5) ein Paar Seitenflügel (16) aufweist, und wobei das Gehäuse (2) und der Verbrauchsteil so ausgebildet sind, dass die Verbrauchskomponente (5) mit dem Gehäuse (2) koppelbar ist, indem dessen Seitenflügel (16) jeweils in die Führungen (15) eingesetzt werden und indem die Verbrauchskomponente (5) auf der Hinterfläche (13) des Gehäuses (2) bewegt wird,
wobei die elektrischen Anschlussmittel (12) innerhalb von den Kopplungsflächen des Paares Führungen (15) und der Verbrauchskomponente (5) umfasst sind,
wobei die Kopplungsflächen des Paares Führungen (15) und die Verbrauchskomponente (5) dazu ausgebildet sind, um sich auf abgedichtete Weise wirksam zu koppeln, so dass sie den elektrischen Anschluss abdichten und
wobei die Fläche der Verbrauchskomponente (5), welche dazu ausgebildet ist, die Fläche des Paares Führungen (15), welche die elektrischen Anschlussmittel (12) umfassen, eine Haftfläche umfasst, welche dazu ausgebildet ist, die Kopplung zusätzlich abzudichten.

2. Tragbare Vorrichtung (1) nach einem der vorhergehenden Ansprüche, wobei ein Teil der Hinterfläche (13) des Gehäuses (2) im Allgemeinen flach ist, und wobei die Verbrauchskomponente (5) mit dem Gehäuse (2) koppelbar und entkoppelbar ist, indem die Verbrauchskomponente (5) auf einer Ebene parallel zum genannten im Allgemeinen flachen Teil der Hinterfläche (13) oder auf einer Ebene komplanar mit der flachen Fläche bewegt wird.

3. Tragbare Vorrichtung (1) nach einem der vorhergehenden Ansprüche, wobei die Verbrauchskomponente (5) im Allgemeinen ein flacher Körper ist.

4. Tragbare Vorrichtung (1) nach einem der vorhergehenden Ansprüche, wobei die Mittel (3) zum Befestigen des Hauptgehäuses (2) an einem Teil des Körpers eines Benutzers einen flexiblen Streifen umfasst, welcher zwei Enden aufweist, welche jeweils mit dem Gehäuse (2) koppelbar sind.

5. Tragbare Vorrichtung (1) nach einem der Ansprüche 1 bis 3, wobei die Mittel (3) zum Befestigen des Hauptgehäuses (2) an einem Teil des Körpers eines Benutzers eine Haftfläche umfasst, welche dafür geeignet ist, auf der Haut eines Benutzers angehaftet zu werden, und wobei die Haftfläche vorzugsweise auf der Kontaktfläche (6) der Verbrauchskomponente vorgesehen ist.

6. Tragbare Vorrichtung (1) nach einem der vorhergehenden Ansprüche, wobei das Gehäuse (2) eine Aushöhlung (8) an dessen Hinterfläche (13), um eine Batterie (9) aufzunehmen, um die Verarbeitungseinheit mit Strom zu versorgen, und einen Deckel (10) zum Schließen der Aushöhlung (8) und zum Einschließen der Batterie (9) darin, aufweist, und wobei die Vorrichtung (1) so ausgebildet ist, dass die Verbrauchskomponente (5) mit dem Deckel (10) überlappt ist, wenn die Verbrauchskomponente (5) mit dem Gehäuse (2) wirksam gekoppelt ist.

7. Tragbare Vorrichtung (1) nach den Ansprüchen 4 oder 6, wenn sie von Anspruch 4 abhängt, wobei das Gehäuse (2) mit einem Paar elektrischer Verbinder (12) versehen ist, und jedes Ende des flexiblen Streifens mit metallischen Verbindern ausgerüstet ist, um den flexiblen Streifen und den Biosensor mit dem Paar elektrischer Verbinder (12) des Gehäuses (2) mechanisch und elektrisch zu verbinden.

8. Tragbare Vorrichtung (1) nach Anspruch 4, wobei der flexible Streifen mit mindestens einem Biosensor versehen ist, welcher dazu angeordnet ist, ein Vitalzeichen oder physiologisches Zeichen des Benutzers zu messen, wenn die Vorrichtung (1) vom Benutzer getragen wird, und wobei der Biosensor aus der folgenden Liste ausgewählt wird: einem Herzfrequenzsensor, einem Atemfrequenzsensor, einem Blutdrucksensor, einem Körpertemperatursensor und einem Sauerstoffsättigungssensor.

9. Tragbare Vorrichtung (1) nach einem der vorhergehenden Ansprüche, wobei die Verbrauchskomponente (5) zusätzlich einen Schweißvolumensensor (18) zum Messen des Volumens des gesammelten Schweißes umfasst, und wobei die Verarbeitungseinheit dazu angepasst ist, Daten, welche vom Schweißsensor, dem Vitalzeichenbiosensor und der Schweißvolumenmessvorrichtung bereitgestellt werden, zu empfangen und zu verarbeiten.

10. Tragbare Vorrichtung (1) nach einem der vorhergehenden Ansprüche, zusätzlich umfassend ein Kommunikationsmodul, welches im Gehäuse (2) eingeschlossen ist und für die drahtlose Übertragung von Daten angepasst ist, welche von der Verarbeitungseinheit verarbeitet werden.

11. Tragbare Vorrichtung (1) nach Anspruch 9, wobei der Schweißsensor ein Schweißlaktatsensor ist, und der Vitalzeichensensor ein Herzfrequenzsensor ist, und wobei die Verarbeitungseinheit zusätzlich dazu angepasst ist, vorzugsweise mittels Algorithmen für maschinelles Lernen, eine Blutlaktatkonzentration zu berechnen oder zu schätzen, basierend auf den Daten, welche von Folgenden bereitgestellt werden: dem Schweißlaktatsensor, dem Schweißvolumensensor (18) und dem Herzfrequenzsensor.

12. Tragbare Vorrichtung (1) nach einem der vorhergehenden Ansprüche, zusätzlich umfassend eine Erfassungskammer (21) und mindestens einen der folgenden Sensoren: einen Schweißlaktatsensor, einen Schweißleitfähigkeitssensor, einen Metabolitsensor, Ionensensoren, einen Aminosäuresensor, und, in der Erfassungskammer (21) platziert, und wobei der mikrofluidische Kanal (22) den Schweißeinlass (11) mit der Erfassungskammer (21) kommuniziert.

13. Tragbare Vorrichtung (1) nach einem der vorhergehenden Ansprüche, wobei der Schweißvolumensensor (18) ein Paar Elektroden (20, 20') und einen mikrofluidischen Vorrat (19) zwischen dem Paar Elektroden (20, 20'), und fluidisch mit der Erfassungskammer (21) kommuniziert und stromabwärts der Erfassungskammer (21) angeordnet umfasst, sodass ein Kapazitätswert zwischen den beiden Elektroden (20, 20') in Abhängigkeit von der Schweißmenge im Vorrat (19) variabel ist.

## Revendications

1. Dispositif portable (1) pour la surveillance continue de paramètres de santé d'un utilisateur, comprenant :
un logement (2) ayant une surface avant et une surface arrière (13), et une unité de traitement enfermé dans celui-ci,
des moyens (3) pour attacher le logement (2) à une partie du corps de l'utilisateur,
un composant consommable (5) configuré pour être couplé et découplé manuellement par rapport au logement (2), et ayant une surface de contact (6) destinée à être en contact avec la peau de l'utilisateur lorsque le composant consommable (5) est opérationnellement couplé avec le logement (2) et le logement (2) est attaché à une partie du corps de l'utilisateur, et
dans lequel le composant consommable (5) comprend :
une entrée de collection de sueur (11) formé dans la surface de contact (6) du composant consommable, pour collecter de la sueur lorsque le dispositif (1) est porté par l'utilisateur,
au moins un capteur pour mesurer un biomarqueur de sueur,
un canal microfluidique (22) pour transporter la sueur collectée de l'entrée (11) vers le capteur de sueur,
**caractérisé en ce que** le dispositif portable (1) comprend en outre :
des moyens de connexion électrique (12) pour connecter électriquement le capteur avec l'unité de traitement, lorsque le composant consommable (5) est opérationnellement couplé avec le logement (2),
dans lequel l'unité de traitement est adaptée pour traiter des données fournis par le capteur,
dans lequel le logement (2) présente une paire de guides (15) opposés l'un à l'autre, et dans lequel le composant consommable (5) présente une paire d'ailes latérales (16), et dans lequel le logement (2) et la partie consommable sont configurés de telle sorte que le composant consommable (5) peut être couplé avec le logement (2) en insérant ses ailes latérales (16), respectivement, dans les guides (15) et en déplaçant le composant consommable (5) sur la surface arrière (13) du logement (2),
dans lequel les moyens de connexion électrique (12) sont compris à l'intérieur des surfaces de couplage de la paire de guides (15) et le composant consommable (5),
dans lequel les surfaces de couplage de la paire de guides (15) et le composant consommable (5) sont configurées pour le couplage opérationnel de manière étanche de sorte qu'elles scellent la connexion électrique et
dans lequel la surface du composant consommable (5) configurée pour entrer en contact avec la surface de la paire de guides (15) comprenant les moyens de connexion électrique (12) comprend une surface adhésive configurée pour sceller davantage le couplage.

2. Dispositif portable (1) selon l'une quelconque des revendications précédentes, dans lequel une partie de la surface arrière (13) du logement (2) est généralement plate, et dans lequel le composant consommable (5) peut être couplé et découplé par rapport au logement (2) en déplaçant le composant consommable (5) sur un plan parallèle à ladite partie généralement plate de la surface arrière (13), ou sur un plan coplanaire avec la surface plate.

3. Dispositif portable (1) selon l'une quelconque des revendications précédentes, dans lequel le composant consommable (5) est généralement un corps plat.

4. Dispositif portable (1) selon l'une quelconque des revendications précédentes, dans lequel les moyens (3) pour attacher le logement principal (2) à une partie du corps d'un utilisateur, comprennent une bande flexible ayant deux extrémités qui peuvent être couplées, respectivement, avec le logement (2).

5. Dispositif portable (1) selon l'une quelconque des revendications 1 à 3, dans lequel les moyens (3) pour attacher le logement principal (2) à une partie du corps d'un utilisateur, comprennent une surface adhésive appropriée pour s'adhérer sur la peau d'un utilisateur, et dans lequel, de préférence, la surface adhésive est prévue sur la surface de contact (6) du composant consommable.

6. Dispositif portable (1) selon l'une quelconque des revendications précédentes, dans lequel le logement (2) présente une cavité (8) dans sa surface arrière (13) pour recevoir une batterie (9) pour alimenter en puissance l'unité de traitement, et un couvercle (10) pour fermer la cavité (8) et enfermer la batterie (9) dans celle-ci, et dans lequel le dispositif (1) est configuré de telle sorte que le couvercle (10) est superposé sur le composant consommable (5), lorsque le composant consommable (5) est opérationnellement couplé avec le logement (2).

7. Dispositif portable (1) selon les revendications 4 ou 6, lorsqu'elles dépendent de la revendication 4, dans lequel le logement (2) est pourvu d'une paire de connecteurs électriques (12), et chaque extrémité de la bande flexible est équipée de connecteurs métalliques pour connecter mécaniquement et électriquement la bande flexible et le biocapteur avec la paire de connecteurs électriques (12) du logement (2).

8. Dispositif portable (1) selon la revendication 4, dans lequel la bande flexible est pourvue d'au moins un biocapteur disposé pour mesurer un signe vital ou un signe physiologique de l'utilisateur, lorsque le dispositif (1) est porté par un utilisateur, et dans lequel le biocapteur est choisi parmi la liste suivante : un capteur de fréquence cardiaque, un capteur de fréquence respiratoire, un capteur de tension artérielle, un capteur de température corporelle, et un capteur de saturation en oxygène.

9. Dispositif portable (1) selon l'une quelconque des revendications précédentes, dans lequel le composant consommable (5) comprend en outre un capteur de volume de sueur (18) pour mesurer le volume de la sueur collectée, et dans lequel l'unité de traitement est adaptée pour recevoir et traiter des données fournis par le capteur de sueur, le biocapteur de signes vitaux et le dispositif de mesure de volume de sueur.

10. Dispositif portable (1) selon l'une quelconque des revendications précédentes, comprenant en outre un module de communication enfermé dans le logement (2), et adapté pour la transmission sans fil de données traités par l'unité de traitement.

11. Dispositif portable (1) selon la revendication 9, dans lequel le capteur de sueur est un capteur de lactate de sueur, et le capteur de signes vitaux est un capteur de fréquence cardiaque, et dans lequel l'unité de traitement est adaptée en outre pour calculer ou estimer, de préférence par le biais d'algorithmes d'apprentissage automatique, une concentration de lactate dans le sang sur la base des données fournis par : le capteur de lactate de sueur, le capteur de volume de sueur (18) et le capteur de fréquence cardiaque.

12. Dispositif portable (1) selon l'une quelconque des revendications précédentes, comprenant en outre une chambre de détection (21) et au moins l'un des capteurs suivants : un capteur de lactate de sueur, un capteur de conductivité de sueur, un capteur de métabolites, des capteurs d'ions, un capteur d'acides aminés, et, placé dans la chambre de détection (21), et dans lequel le canal microfluidique (22) met l'entrée de sueur (11) en communication avec la chambre de détection (21).

13. Dispositif portable (1) selon l'une quelconque des revendications précédentes, dans lequel le capteur de volume de sueur (18) comprend une paire d'électrodes (20, 20') et un réservoir microfluidique (19) entre la paire d'électrodes (20, 20'), et en communication de fluide avec la chambre de détection (21) et disposé en aval de la chambre de détection (21), de telle sorte qu'une valeur de capacité entre les deux électrodes (20, 20') est variable en fonction de la quantité de sueur dans le réservoir (19).
